# FASCICULE DE BREVET EUROPEEN

(11) **EP 3 280 327 B1**
(45) Date de publication et mention de la délivrance du brevet: **14.08.2024**
(21) Numéro de dépôt: 16720882.6
(22) Date de dépôt: 06.04.2016
(51) Int. Cl.: A61B 5/053, A61B 5/00, A61B 17/70

(54) **SYSTÈME MÉDICAL**
MEDIZINISCHES SYSTEM
MEDICAL SYSTEM

(30) Priorité: 07.04.2015 FR 1552988
(43) Date de publication de la demande: 14.02.2018
(73) Titulaire: Spineguard, 94300 Vincennes (FR)
(72) Inventeur: BOURLION, Maurice, 42800 Rive De Gier (FR); BOLGER, Ciaran, Dublin (IE); FREZAL, Olivier, 93110 Rosny Sous Bois (FR); BETTE, Stéphane, Corte Madera, CA 94925 (US)
(74) Mandataire: Icosa
(86) Numéro de dépôt international: PCT/FR2016/050789
(87) Numéro de publication internationale: WO 2016/162634

(56) Documents cités:
- WO-A1-2012/066231
- FR-A1- 2 865 922
- US-A- 5 902 105
- US-A1- 2005 119 660

## Description

L'invention se rapporte à un système médical.

En particulier, l'invention se rapporte à un système médical destiné à pénétrer dans une structure anatomique d'un patient, laquelle structure anatomique comporte des tissus présentant des capacités à conduire un courant électrique différentes. Le système médical est du type comprenant :
- un corps adapté pour pénétrer dans la structure anatomique, le corps présentant une surface extérieure,
- au moins une première électrode comportant une première surface de contact agencée sur la surface extérieure du corps pour venir en contact avec les tissus de la structure anatomique,
- au moins une deuxième électrode comportant une deuxième surface de contact agencée sur la surface extérieure du corps pour venir en contact avec les tissus de la structure anatomique à distance de la première surface de contact,
- un générateur électrique adapté pour appliquer un courant électrique entre les première et deuxième surfaces de contact,
- un dispositif de traitement adapté pour déterminer une caractéristique électrique représentative de la capacité du tissu de la structure anatomique entre les première et deuxième surfaces de contact à conduire le courant électrique, et pour émettre un signal d'avertissement correspondant à la caractéristique électrique déterminée, le signal d'avertissement présentant au moins un paramètre variable en fonction de la caractéristique électrique déterminée.

Les documents FR 2 865 922 et WO 03/068076 décrivent un système médical du type précité sous la forme d'un instrument chirurgical dans lequel le signal d'avertissement est intermittent et présente une cadence d'avertissement selon laquelle des sections d'avertissement perceptibles par un utilisateur sont émises successivement avec un laps de temps entre deux sections d'avertissement successives.

Cet instrument chirurgical commercialisé sous le nom de PediGuard ^{®} est notamment utilisé en chirurgie orthopédique pour assurer le positionnement approprié de vis pédiculaires dans les pédicules vertébraux d'une vertèbre d'un patient pour la fixation d'une prothèse ou d'un implant. Il est, en effet, important d'assurer un positionnement précis des vis pédiculaires dans l'os spongieux des pédicules vertébraux pour ancrer de manière satisfaisante la prothèse ou l'implant tout en évitant d'endommager ou pire de traverser la couche d'os cortical interne délimitant le foramen vertébral dans lequel passe la moelle épinière ou la couche d'os corticale externe au voisinage de laquelle passent les racines nerveuses. Les variations du signal d'avertissement fournissent une information sur les tissus au voisinage des première et deuxième surfaces de contact, l'os cortical présentant une conductivité électrique inférieure à celle de l'os spongieux qui présente lui-même une conductivité électrique inférieure à celle de fluides, tels que du sang, ou celle de tissus mous.

L'instrument chirurgical dont l'utilisation est simple et intuitive donne entière satisfaction et offre des taux de réussite spectaculaires quant au placement des vis pédiculaires.

Néanmoins, dans certaines situations particulières et notamment lorsque les tissus rencontrés présentent de nombreuses inhomogénéités locales, la sensibilité de l'instrument chirurgical à des changements très ponctuels dans la capacité à conduire le courant électrique peut conduire à certaines difficultés d'interprétation du signal d'avertissement. En particulier, ces changements ponctuels peuvent se traduire par des variations intempestives du signal d'avertissement interrompant une succession attendue par le praticien de sections d'avertissement et de laps de temps. Des changements dans la capacité à conduire le courant électrique intervenant en nombre dans un court intervalle de temps peuvent également conduire à des artéfacts dans le signal d'avertissement fournissant au praticien une information inconnue ou difficile à interpréter.

Dans ces situations particulières, la bonne utilisation de l'instrument chirurgical peut reposer sur l'expérience du praticien pour discerner les informations dont il doit tenir compte.

L'invention vise à pallier ce problème en améliorant la fiabilité du système médical qu'elle qu'en soit l'utilisation.

A cet effet, l'invention propose un système médical tel que défini dans la revendication 1.

Ainsi, l'invention prévoit qu'une période définie par la temporisation précède l'émission du signal d'avertissement avec le ou les paramètres correspondant à la caractéristique électrique déterminée. L'émission du signal d'avertissement avec le ou les paramètres correspondant à la caractéristique électrique déterminée est donc différée par rapport à la détection de la variation de la caractéristique électrique. La temporisation permet alors de limiter ou de moduler la sensibilité du système médical aux changements ponctuels dans la capacité des tissus rencontrés à conduire le courant électrique. Les variations intempestives du signal d'avertissement et les risques d'artéfacts s'en trouvent réduits améliorant ainsi la fiabilité du système médical.

La cadence d'avertissement peut être comprise entre 1 Hz et 20 Hz.

Ledit au moins un paramètre variable du signal d'avertissement peut comprendre une fréquence d'avertissement à laquelle chacune des sections d'avertissement est émise, le dispositif de traitement étant adapté pour modifier la fréquence d'avertissement après que la temporisation est écoulée.

La fréquence d'avertissement peut être comprise entre 470 Hz et 2600 Hz.

Ledit au moins un paramètre variable du signal d'avertissement peut comprendre une amplitude d'avertissement, le dispositif de traitement étant adapté pour modifier l'amplitude d'avertissement après que la temporisation est écoulée.

Le dispositif de traitement peut être adapté pour déterminer une conductivité électrique comme caractéristique électrique, et pour :
- augmenter le paramètre du signal d'avertissement lorsque la conductivité électrique augmente,
- diminuer le paramètre du signal d'avertissement lorsque la conductivité électrique diminue.

De façon alternative, le dispositif de traitement peut être adapté pour déterminer une résistivité électrique comme caractéristique électrique, et pour :
- augmenter le paramètre du signal d'avertissement lorsque la résistivité électrique diminue,
- diminuer le paramètre du signal d'avertissement lorsque la résistivité électrique augmente.

Le dispositif de traitement peut être adapté pour maintenir constant ledit au moins un paramètre variable du signal d'avertissement tant que la caractéristique électrique est inférieure à un seuil, et pour faire varier le paramètre du signal d'avertissement lorsque la caractéristique électrique atteint le seuil.

D'autres objets et avantages de l'invention apparaîtront à la lecture de la description qui suit d'un mode de réalisation particulier de l'invention donné à titre d'exemple non limitatif, la description étant faite en référence aux dessins annexés dans lesquels :

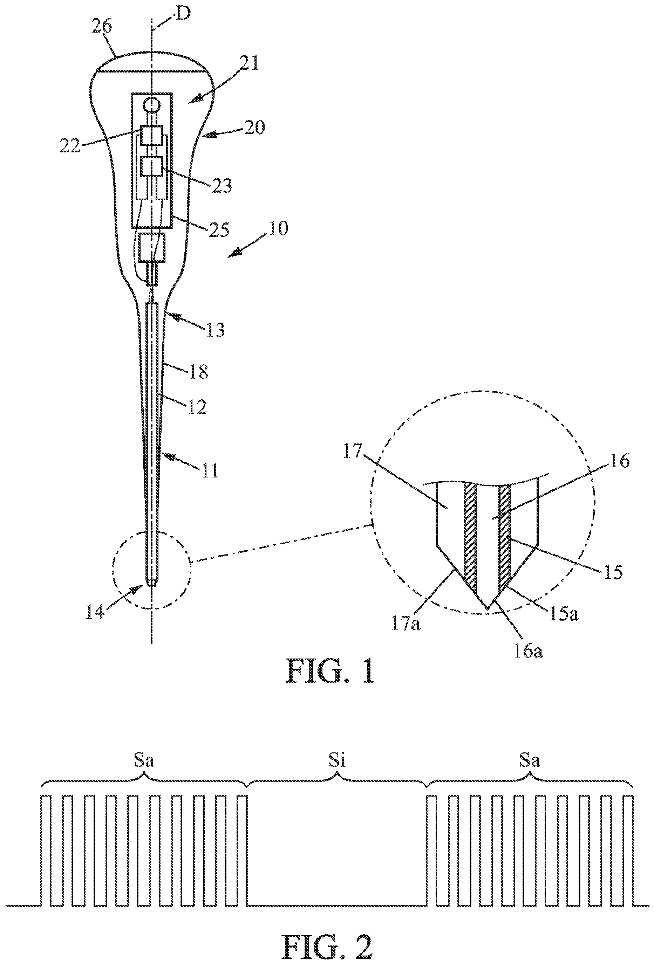

- la figure 1 est une représentation schématique d'un système médical selon un mode de réalisation de l'invention, le système médical comprenant un dispositif de traitement adapté pour émettre un signal d'avertissement correspondant à une caractéristique électrique représentative de la capacité d'un tissu d'une structure anatomique entre des première et deuxième surfaces de contact à conduire un courant électrique,
- la figure 2 est une représentation du signal d'avertissement intermittent et présentant une cadence d'avertissement selon laquelle des sections d'avertissement perceptibles par un utilisateur sont émises successivement avec un laps de temps entre deux sections d'avertissement successives, chaque section d'avertissement présentant une fréquence d'avertissement et une amplitude d'avertissement,

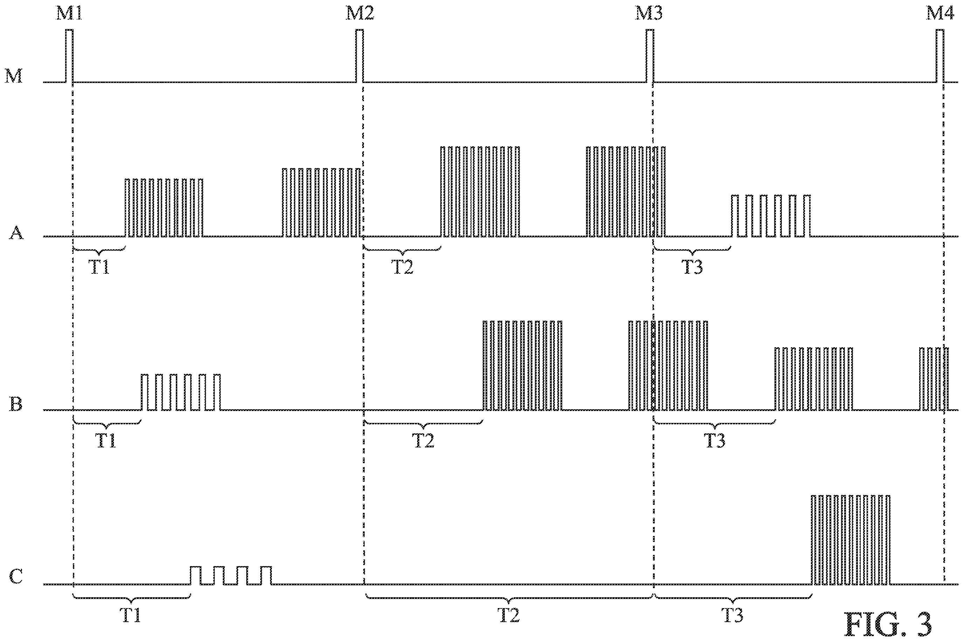

- la figure 3 est une représentation d'un signal de mesure (M) et de trois signaux d'avertissement (A, B, C) émis respectivement au cours de la pénétration du système médical dans trois structures anatomiques différentes, la cadence d'avertissement, la fréquence d'avertissement et l'amplitude d'avertissement variant après qu'une temporisation suivant la variation de la caractéristique électrique est écoulée,

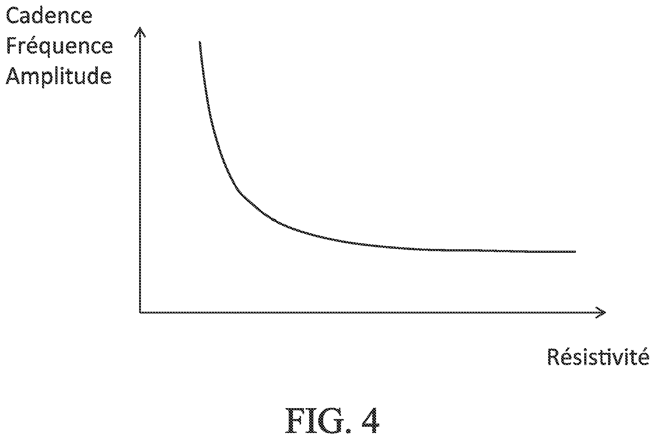

- la figure 4 est une représentation d'une fonction de transfert enregistrée dans le dispositif de traitement et associant une valeur de cadence d'avertissement, de fréquence d'avertissement et/ou d'amplitude d'avertissement à chacune des valeurs de la caractéristique électrique,

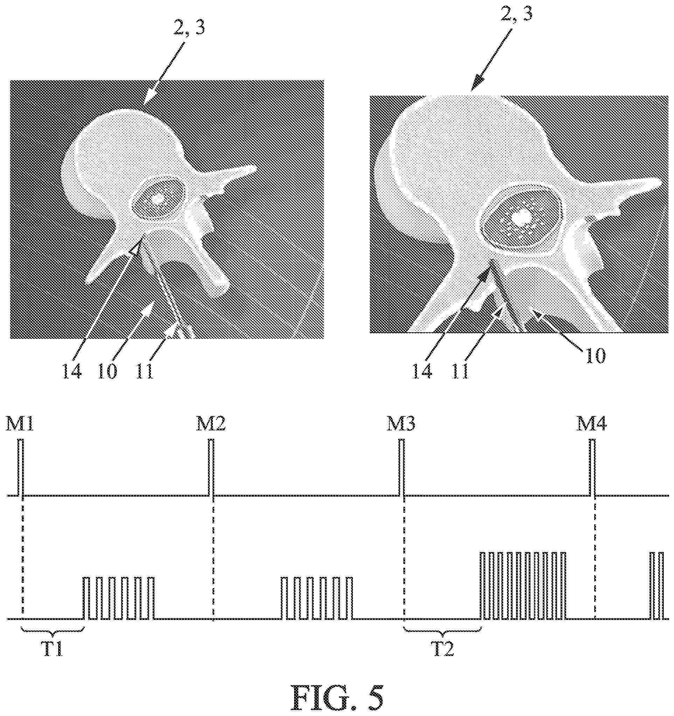

- les figures 5, 6 et 7 sont des représentations du signal de mesure et du signal d'avertissement au cours de la pénétration du système médical dans une vertèbre de la colonne vertébrale d'un patient.

Sur les figures, les mêmes références désignent des éléments identiques ou analogues.

La figure 1 représente schématiquement un système médical sous la forme d'un instrument chirurgical 10 manipulable à la main par un praticien pour forer une structure osseuse 3, telle qu'une vertèbre 2 de la colonne vertébrale d'un patient. L'invention n'est toutefois pas limitée à ce type d'instrument chirurgical, ni à une application à une structure osseuse. En particulier, l'invention peut être mise en œuvre sur tout type de structure anatomique avec tout type de système médical manipulable à la main ou par l'intermédiaire d'un bras robotisé. Le système médical peut comprendre tout type d'instrument ou d'outil chirurgical ou à usage médical, manuel ou motorisé, et, en particulier, une sonde, une pointe carrée, une mèche de perceuse, une spatule ou une curette. Il peut également comprendre un implant tel qu'une vis et, en particulier, une vis pédiculaire.

L'outil 10 comprend un corps 11 adapté pour pénétrer dans la structure osseuse 3, et un boîtier 20 formant une poignée solidarisée au corps 11 et adaptée pour être tenue par la main du praticien. Selon les applications, le boîtier 20 peut également être adapté pour être solidarisé à une extrémité d'un bras de robot.

Le corps 11 présente une surface extérieure 12 et sert de support à des première 16 et deuxième 17 électrodes présentant respectivement des première 16a et deuxième 17a surfaces de contact agencées pour venir en contact avec la structure osseuse 3 à distance l'une de l'autre.

Dans le mode de réalisation représenté, le corps 11 est conique de section circulaire selon un axe central D et s'étend depuis une extrémité proximale 13 solidarisée, éventuellement de manière amovible à la poignée 20 jusqu'à une extrémité distale 14 définissant une extrémité de pénétration, tronconique ou pyramidale. Le corps 11 pourrait, toutefois, présenter toute autre forme, notamment conique ou cylindrique de section polygonale ou autre.

La première électrode 16, cylindrique et en matériau conducteur, s'étend à l'intérieur du corps 11 parallèlement à l'axe central D. En particulier, la première électrode 16 est disposée dans un alésage central du corps 11 et s'étend coaxialement à l'axe central D jusqu'à une extrémité libre formant la première surface de contact 16a. La première surface de contact 16a affleure la surface extérieure 12 du corps 11 à son extrémité distale 14.

La deuxième électrode 17, annulaire et en matériau conducteur, s'étend selon l'axe central D autour de la première électrode 16. La deuxième électrode 17 peut, en particulier, être formée par le corps 11 lui-même, alors réalisé en un matériau conducteur. La deuxième surface de contact 17a de la deuxième électrode 17 est composée d'une portion cylindrique parallèle à l'axe central D correspondant à une surface latérale du corps 11, et une portion annulaire transversale par rapport à l'axe central D correspondant à une surface distale du corps 11.

Une couche de matériau électriquement isolant 15 est interposée entre les première 16 et deuxième 17 électrodes. La couche de matériau électriquement isolant 15 s'étend le long du corps 11, depuis l'extrémité proximale 13 du corps 11 jusqu'à l'extrémité distale 14 du corps 11 à laquelle elle affleure par une surface libre d'extrémité 15a. Dans le mode de réalisation représenté, la couche de matériau électriquement isolant 15, annulaire, s'étendant selon l'axe central D autour de la première électrode 16 et à l'intérieur de la deuxième électrode 17.

L'invention n'est toutefois pas limitée à la réalisation et à l'agencement précédemment décrits du corps 11, des première 16 et deuxième 17 électrodes et de la couche de matériau électriquement isolant 15. De manière plus générale, les première 16 et deuxième 17 électrodes ne sont pas nécessairement agencées de manière coaxiale. En particulier, ces première 16 et deuxième 17 électrodes peuvent chacune être réalisées par une tige en matériau conducteur plongée dans le corps 11. Par ailleurs, la première électrode 16 et la deuxième électrode 17 peuvent chacune présenter une surface de contact 16a, 17a ponctuelle affleurant la surface latérale ou la surface distale du corps 11. Le corps 11 peut en outre supporter deux ou plus de deux premières électrodes 16 et deux ou plus de deux deuxièmes électrodes 17.

La poignée 20, cylindrique de révolution, s'étend sensiblement coaxialement à l'axe central D du corps 11. La poignée 20 présente une forme facilitant la prise en main et la manipulation de l'outil 10. La poignée 20 réalisée en matériau plastique est solidaire d'un manchon 18 de matière plastique s'étendant sur une partie de la surface extérieure 12 du corps 11.

La poignée 20 comporte un logement 21 adapté pour recevoir un générateur électrique 22 et un dispositif de traitement 23, par exemple, placés sur une carte électronique 25 insérée dans le logement 21 au travers d'une ouverture prévue à une extrémité de la poignée 20 opposée au corps 11. Un capot 26 amovible permet de fermer le logement 21.

Le générateur électrique 22 est adapté pour appliquer un courant électrique M entre les première 16a et deuxième 17a surfaces de contact. Dans un mode de réalisation particulier représenté sur la figure 3, sans y être limité, le générateur électrique émet le courant électrique M sous la forme d'impulsions de tension 1,2 V, à une fréquence de mesure de 5 Hz. Le courant électrique M présente alors une période de mesure correspondant à la fréquence de mesure de 200 ms. En variante la tension du courant électrique pourrait être toute tension inférieure à 2 V, de préférence entre 1 V et 2 V, notamment entre 1,1 V et 1,5 V. La fréquence de mesure pourrait être comprise entre 4 Hz et 20 Hz, la période de mesure étant comprise entre 50 ms et 250 ms.

Le dispositif de traitement 23 est alors adapté pour déterminer une caractéristique électrique représentative de la capacité du tissu de la structure osseuse 3 entre les première 16a et deuxième 17a surfaces de contact à conduire le courant électrique M. En particulier, à partir de la tension du courant électrique M, le dispositif de traitement 23 est adapté pour mesurer l'intensité du courant électrique M traversant le tissu entre les première 16a et deuxième 17a surfaces de contact. A partir de la tension connue et de l'intensité mesurée du courant électrique, le dispositif de traitement 23 peut déterminer la caractéristique électrique telle que la résistivité électrique. Cette mesure de l'intensité du courant électrique M et la détermination de la résistivité électrique peuvent être réalisées selon la fréquence de mesure, une mesure étant réalisée à chaque impulsion du courant électrique M. En variante, le générateur électrique 22 pourrait délivrer un courant électrique M dont l'intensité est connue et le dispositif de traitement 23 pourrait être adapté pour mesurer la tension du courant électrique afin de déterminer la caractéristique électrique à partir de l'intensité connue et de la tension mesurée du courant électrique.

Les tissus de la structure osseuse 3 présentent des capacités à conduire le courant électrique différentes. Par exemple, l'os cortical présente une résistivité électrique supérieure à celle de l'os spongieux qui présente lui-même une résistivité électrique supérieure à celle de fluides tels que du sang. Un tel dispositif de traitement 23 permet de détecter, de manière relative, un changement de tissu à partir d'une variation de la résistivité électrique, voire d'identifier, de manière absolue, un tissu à partir d'une valeur de la résistivité électrique.

Le dispositif de traitement 23 est également adapté pour émettre un signal d'avertissement correspondant à la résistivité électrique déterminée. Le signal d'avertissement peut être l'un d'un signal d'avertissement sonore, un signal d'avertissement lumineux et un signal d'avertissement tactile (vibrations) ou une combinaison de tels signaux d'avertissement.

Sur la figure 2, le signal d'avertissement, par exemple sonore, est intermittent, Il présente une cadence d'avertissement selon laquelle des sections d'avertissement Sa perceptibles par le praticien sont émises successivement avec un laps de temps Si entre deux sections d'avertissement Sa successives. La cadence d'avertissement peut notamment être comprise entre 1 Hz et 20 Hz. La cadence d'avertissement est représentative d'une vitesse à la laquelle les sections d'avertissement Sa, correspondant par exemple à des bips dans le cas d'un signal d'avertissement sonore, sont émis. Dans un mode de réalisation particulier, chaque section d'avertissement Sa présente une même durée par exemple de 35,5 ms, le laps de temps Si correspondant à un temps de silence variant en fonction de la cadence d'avertissement. On définit également une période d'avertissement correspondant à la cadence d'avertissement et comprenant une section d'avertissement Sa et un laps de temps Si.

En outre, chaque section d'avertissement Sa est périodique et présente une fréquence d'avertissement. La fréquence d'avertissement peut notamment être comprise entre 470 Hz et 2600 Hz. La fréquence d'avertissement est représentative d'une tonalité de chacune des sections d'avertissement Sa, la section d'avertissement passant de grave à aigu en augmentant la fréquence d'avertissement. Dans un mode de réalisation particulier, chaque section d'avertissement comporte une série d'impulsions ayant chacune une même durée, par exemple de 230 µs.

Chaque section d'avertissement Sa peut également présenter une amplitude d'avertissement représentative de l'intensité à laquelle la section d'avertissement Sa est émise.

Dans le mode de réalisation représenté sur la figure 3, le dispositif de traitement 23 est adapté pour faire varier l'ensemble des paramètres du signal d'avertissement, à savoir la cadence d'avertissement, la fréquence d'avertissement et l'amplitude d'avertissement, en fonction de la résistivité électrique. En particulier, le dispositif de traitement 23 :
- augmente la cadence d'avertissement, la fréquence d'avertissement et l'amplitude d'avertissement du signal d'avertissement lorsque la résistivité électrique diminue, et
- diminue la cadence d'avertissement, la fréquence d'avertissement et l'amplitude d'avertissement lorsque la résistivité électrique augmente.

En variante, le dispositif de traitement 23 pourrait être adapté pour déterminer une conductivité électrique comme caractéristique électrique, et pour :
- augmenter un ou plusieurs des paramètres choisis parmi la cadence d'avertissement, la fréquence d'avertissement et l'amplitude d'avertissement lorsque la conductivité électrique augmente,
- diminuer un ou plusieurs des paramètres choisis parmi la cadence d'avertissement, la fréquence d'avertissement et l'amplitude d'avertissement lorsque la conductivité électrique diminue.

Par ailleurs, comme il ressort de ce qui précède, seul l'un ou deux des paramètres choisis parmi la cadence d'avertissement, la fréquence d'avertissement et l'amplitude d'avertissement pourraient être variables en fonction de la caractéristique électrique déterminée.

En outre, comme il apparaît sur la figure 3, une fois la variation de la résistivité électrique détectée, le dispositif de traitement 23 ne fait varier le ou les paramètres du signal d'avertissement qu'après qu'une temporisation T est écoulée. Cette temporisation peut être choisie de toute manière appropriée pour éviter les perturbations du signal d'avertissement. Elle peut être fixe ou variable.

Sur la figure 3, quatre mesures sont réalisées successivement dans trois structures anatomiques différentes. Après la première mesure, une première temporisation T1 peut être choisie de manière aléatoire, notamment comme une partie de la période de mesure. Pour les mesures suivantes, les temporisations correspondent à des parties non écoulées de la période d'avertissement du signal d'avertissement correspondant à la résistivité électrique déterminée à la mesure précédente.

Ainsi, au cours de la pénétration du corps 11 dans une première structure anatomique, un premier signal d'avertissement A est émis. A une première mesure M1, le dispositif de traitement 23 détermine une première résistivité électrique. Après une première temporisation T1 aléatoire, il émet le signal d'avertissement avec des premières cadence d'avertissement, fréquence d'avertissement et amplitude d'avertissement correspondantes. A une deuxième mesure M2, le dispositif de traitement 23 détermine une deuxième résistivité électrique inférieure à la première résistivité électrique. Après une deuxième temporisation T2 correspondant sensiblement au laps de temps du signal d'avertissement de la première mesure, il émet le signal d'avertissement avec des deuxièmes cadence d'avertissement, fréquence d'avertissement et amplitude d'avertissement correspondantes, supérieures aux premières cadence d'avertissement, fréquence d'avertissement et amplitude d'avertissement. A une troisième mesure M3, le dispositif de traitement 23 détermine une troisième résistivité électrique supérieure aux première et deuxième résistivités électriques. Après une troisième temporisation T3 correspondant à une partie de la période d'avertissement comprenant des impulsions de la section d'avertissement et le laps de temps du signal d'avertissement de la deuxième mesure, il émet le signal d'avertissement avec des troisièmes cadence d'avertissement, fréquence d'avertissement et amplitude d'avertissement correspondantes, inférieures aux premières et deuxièmes cadence d'avertissement, fréquence d'avertissement et amplitude d'avertissement.

De la même manière, au cours de la pénétration du corps 11 dans une deuxième structure anatomique, un deuxième signal d'avertissement B est émis. A la première mesure M1, le dispositif de traitement 23détermine une première résistivité électrique. Après une première temporisation T1 aléatoire, il émet le signal d'avertissement avec des premières cadence d'avertissement, fréquence d'avertissement et amplitude d'avertissement correspondantes. A la deuxième mesure M2, le dispositif de traitement 23détermine une deuxième résistivité électrique inférieure à la première résistivité électrique. Après une deuxième temporisation T2 correspondant à une partie du laps de temps du signal d'avertissement de la première mesure, il émet le signal d'avertissement avec des deuxièmes cadence d'avertissement, fréquence d'avertissement et amplitude d'avertissement correspondantes, supérieures aux premières cadence d'avertissement, fréquence d'avertissement et amplitude d'avertissement. A la troisième mesure M3, le dispositif de traitement 23 détermine une troisième résistivité électrique inférieure à la première résistivité électrique et supérieure à la deuxième résistivité électrique. Après une troisième temporisation T3 correspondant sensiblement à une partie de la période d'avertissement comprenant des impulsions de la section d'avertissement et le laps de temps du signal d'avertissement de la deuxième mesure, il émet le signal d'avertissement avec des troisièmes cadence d'avertissement, fréquence d'avertissement et amplitude d'avertissement correspondantes, supérieures aux premières cadence d'avertissement, fréquence d'avertissement et amplitude d'avertissement et inférieures aux deuxièmes cadence d'avertissement, fréquence d'avertissement et amplitude d'avertissement.

Au cours de la pénétration du corps 11 dans une troisième structure anatomique, un troisième signal d'avertissement C est émis. A la première mesure M1, le dispositif de traitement 23détermine une première résistivité électrique. Après une première temporisation T1 aléatoire, il émet le signal d'avertissement avec des premières cadence d'avertissement, fréquence d'avertissement et amplitude d'avertissement correspondantes. A la deuxième mesure M2, le dispositif de traitement 23 détermine une deuxième résistivité électrique. Toutefois, cette mesure intervient au cours d'une deuxième temporisation correspondant à une partie du laps de temps de la première mesure. Aucun signal d'avertissement correspondant à la deuxième mesure n'est émis avant que la troisième mesure M3 ne soit réalisée. A la troisième mesure M3, le dispositif de traitement 23détermine une troisième résistivité électrique inférieure à la première résistivité électrique. Après une troisième temporisation T3 correspondant à une partie du laps de temps de la première mesure, il émet le signal d'avertissement avec des deuxièmes cadence d'avertissement, fréquence d'avertissement et amplitude d'avertissement correspondantes, supérieures aux premières cadence d'avertissement, fréquence d'avertissement et amplitude d'avertissement.

En variante, la temporisation T pourrait être choisie de toute autre manière appropriée. La temporisation peut notamment être comprise entre 30% et 100% du laps de temps Si, notamment entre 50% et 100% du laps de temps Si, et par exemple entre 60% et 90% du laps de temps Si.

Selon une autre variante, la temporisation peut être égale à au moins une partie de la période de mesure, de préférence comprise entre 10% et 500% de la période de mesure. Dans cette autre variante, lorsque la temporisation est supérieure à deux fois la période de mesure, le dispositif de traitement peut être adapté pour calculer une caractéristique électrique moyenne à partir des caractéristiques électriques déterminées à chacune des périodes de mesure intervenues au cours de la temporisation. Le ou les paramètres du signal d'avertissement peuvent alors être modulés en fonction de la caractéristique électrique moyenne calculée. Ces dispositions permettent de limiter la sensibilité de l'instrument chirurgical 10 aux inhomogénéités locales.

En relation avec les figures 4 à 7, la mise en œuvre de l'instrument chirurgical 10 au cours de la formation d'un trou dans l'un des pédicules vertébraux est décrite. L'os cortical présente une résistivité électrique supérieure à celle de l'os spongieux et l'os spongieux présente une résistivité électrique supérieure à celle du sang.

La figure 4 représente un exemple de fonction de transfert enregistrée dans le dispositif de traitement 23 et associant une valeur de cadence d'avertissement, de fréquence d'avertissement et/ou d'amplitude d'avertissement à chacune des valeurs de la résistivité électrique. Lorsque le tissu avec lequel les première 16a et deuxième 17a surfaces de contact de l'instrument chirurgical 10 sont en contact change, il se produit une variation de résistivité électrique qui se traduit par une variation d'au moins l'un des paramètres parmi la cadence d'avertissement, la fréquence d'avertissement et l'amplitude d'avertissement.

La fonction de transfert est choisie pour diminuer les cadence d'avertissement, fréquence d'avertissement et/ou amplitude d'avertissement au fur et à mesure que la résistivité électrique augmente.

De cette manière, lorsque les première 16a et deuxième 17a surfaces de contact de l'instrument chirurgical 10 sont en contact avec le sang ou les tissus mous dont la résistivité électrique est basse, le signal d'avertissement a des cadence d'avertissement, fréquence d'avertissement et amplitude d'avertissement élevées. Lorsque les première 16a et deuxième 17a surfaces de contact de l'instrument chirurgical 10 sont en contact avec l'os spongieux dont la résistivité électrique est entre celle du sang ou des tissus mous et celle de l'os cortical, le signal d'avertissement a des cadence d'avertissement, fréquence d'avertissement et amplitude d'avertissement intermédiaires. Et lorsque les première 16a et deuxième 17a surfaces de contact de l'instrument chirurgical 10 sont en contact avec l'os cortical dont la résistivité électrique est élevée, le signal d'avertissement a des cadence d'avertissement, fréquence d'avertissement et/ou amplitude d'avertissement basses.

La fonction de transfert est également choisie pour faire varier les cadence d'avertissement, fréquence d'avertissement et/ou amplitude d'avertissement de façon plus importante pour les résistivités électriques faibles que pour les résistivités électriques élevées.

De cette manière, l'instrument chirurgical permet de traduire de manière plus sensible les variations de résistivité électrique dans les situations présentant les plus de risque pour le patient, à savoir lorsque les première 16a et deuxième 17a surfaces de contact de l'instrument chirurgical 10 sont en contact ou à proximité du sang ou de tissus mous.

Sur la figure 5, deux premières mesures M1, M2 sont réalisées alors que l'extrémité distale 14 du corps 11 de l'instrument chirurgical 10 portant les première 16a et deuxième 17a surfaces de contact est en contact avec la couche externe d'os cortical. Après une première temporisation T1 suivant la première mesure M1, le premier signal d'avertissement correspondant à la résistivité électrique de l'os cortical est émis et continue à l'être après la deuxième mesure M2, aucune variation de la résistivité électrique n'ayant été détectée. A la troisième mesure M3, l'extrémité distale 14 du corps 11 de l'instrument chirurgical 10 a pénétré l'os spongieux. Après une deuxième temporisation T2 suivant la troisième mesure M3, le deuxième signal d'avertissement correspondant à la résistivité électrique de l'os spongieux est émis et continue à l'être après la quatrième mesure M4, aucune variation de la résistivité électrique n'ayant été détectée.

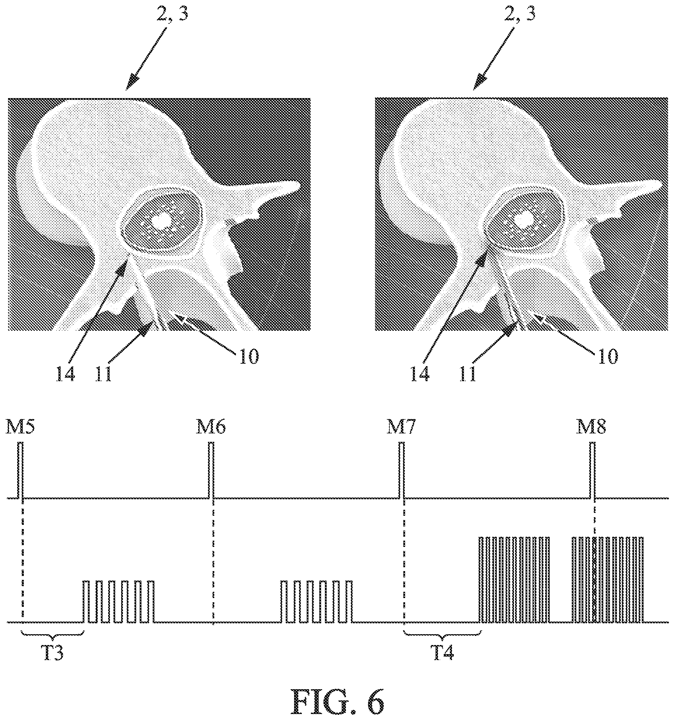

Sur la figure 6, à la cinquième mesure M5, l'extrémité distale 14 du corps 11 de l'instrument chirurgical 10 est à proximité de la couche interne d'os cortical bordant le foramen vertébral. Après une troisième temporisation T3 suivant la cinquième mesure M5, le premier signal d'avertissement correspondant à la résistivité électrique de l'os cortical est émis et continue à l'être après la sixième mesure M6, aucune variation de la résistivité électrique n'ayant été détectée. A la septième mesure M7, l'extrémité distale 14 du corps 11 de l'instrument chirurgical 10 a créé une brèche dans l'os cortical de sorte que du sang s'infiltre dans la cavité formée par le corps 11. Après une quatrième temporisation T4 suivant la septième mesure M7, le troisième signal d'avertissement correspondant à la résistivité électrique du sang est émis et continue à l'être après la huitième mesure M8, aucune variation de la résistivité électrique n'ayant été détectée.

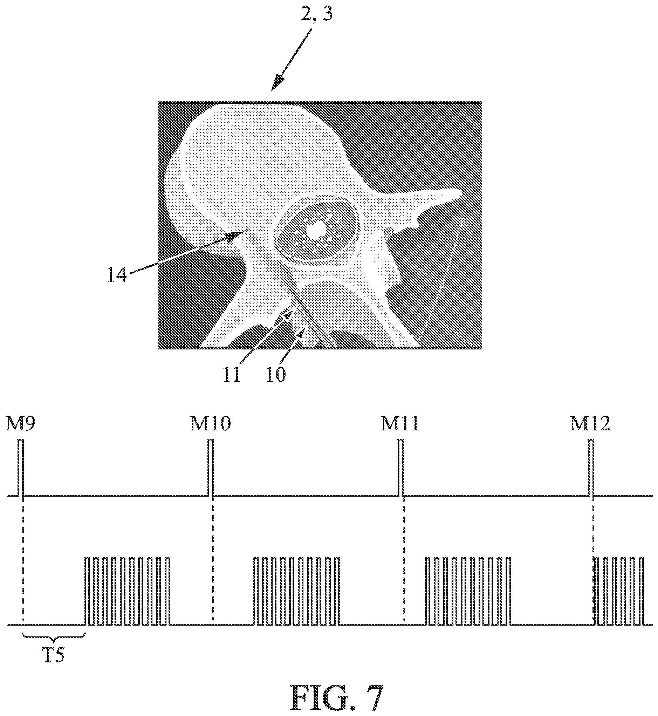

Sur la figure 7, avant la neuvième mesure M9, le praticien a corrigé la trajectoire suite à la perception du troisième signal d'avertissement. L'extrémité distale 14 du corps 11 de l'instrument chirurgical 10 a été ramenée dans l'os spongieux de sorte qu'après une cinquième temporisation T5 suivant la neuvième mesure M9, le deuxième signal d'avertissement correspondant à la résistivité électrique de l'os spongieux est émis et continue à l'être après les dixième M10, onzième M11 et douzième M12 mesures, aucune variation de la résistivité électrique n'ayant été détectée.

En variante, pour limiter la sensibilité de l'instrument chirurgical aux inhomogénéités locales, des plages de résistivité électrique peuvent être définies à partir de seuils. Des paramètres correspondants du signal d'avertissement peuvent être choisis pour chaque plage de résistivité électrique. Le ou les paramètres du signal d'avertissement restent alors constants tant que la résistivité électrique déterminée est dans une plage déterminée, inférieure à un certain seuil. Lorsque la résistivité électrique déterminée change de plage de résistivité électrique et dépasse le seuil concerné, un ou plusieurs des paramètres choisis parmi la cadence d'avertissement, la fréquence d'avertissement et l'amplitude d'avertissement peuvent varier.

En particulier, avec un instrument chirurgical analogue à l'instrument chirurgical décrit précédemment mais permettant de déterminer une valeur absolue de la résistivité électrique à la place d'une simple variation de cette résistivité électrique, trois plages de résistivité électrique pourraient être définies. On pourrait ainsi définir :
- une première plage de résistivité électrique pour l'os cortical à laquelle serait associé un premier signal d'avertissement avec des cadence d'avertissement, fréquence d'avertissement et amplitude d'avertissement basses et constantes,
- une deuxième plage de résistivité électrique pour l'os spongieux à laquelle serait associé un deuxième signal d'avertissement avec des cadence d'avertissement, fréquence d'avertissement et amplitude d'avertissement intermédiaires et constantes,
- une troisième plage de résistivité électrique pour le sang ou les tissus mous à laquelle serait associé un troisième signal d'avertissement avec des cadence d'avertissement, fréquence d'avertissement et amplitude d'avertissement élevées et constantes.

## Revendications

1. Système médical (10) destiné à pénétrer dans une structure anatomique (2, 3) d'un patient, la structure anatomique (2, 3) comportant des tissus présentant des capacités à conduire un courant électrique différentes, ledit système médical (10) comprenant :
- un corps (11) adapté pour pénétrer dans la structure anatomique (2, 3), le corps (11) présentant une surface extérieure (12),
- au moins une première électrode (16) comportant une première surface de contact (16a) agencée sur la surface extérieure (12) du corps (11) pour venir en contact avec les tissus de la structure anatomique (2, 3),
- au moins une deuxième électrode (17) comportant une deuxième surface de contact (17a) agencée sur la surface extérieure (12) du corps (11) pour venir en contact avec les tissus de la structure anatomique (2, 3) à distance de la première surface de contact (16a),
- un générateur électrique (22) adapté pour appliquer un courant électrique (M) entre les première (16a) et deuxième (17a) surfaces de contact,
- un dispositif de traitement (23) adapté pour déterminer une caractéristique électrique représentative de la capacité du tissu de la structure anatomique (2, 3) entre les première (16a) et deuxième (17a) surfaces de contact à conduire le courant électrique (M), et pour émettre un signal d'avertissement correspondant à la caractéristique électrique déterminée, le signal d'avertissement présentant au moins un paramètre variable en fonction de la caractéristique électrique déterminée,
dans lequel le signal d'avertissement est intermittent et présente une cadence d'avertissement selon laquelle des sections d'avertissement (Sa) perceptibles par un utilisateur sont émises successivement avec un laps de temps (Si) entre deux sections d'avertissement (Sa) successives,
dans lequel le dispositif de traitement (23) est adapté pour détecter une variation de la caractéristique électrique et pour faire varier ledit au moins un paramètre variable du signal d'avertissement après qu'une temporisation (T) suivant la variation de la caractéristique électrique est écoulée,
le système médical (10) étant **caractérisé en ce que** la temporisation (T) est égale à une partie d'une période d'avertissement correspondant à la cadence d'avertissement et comprenant une section d'avertissement (Sa) et un laps de temps (Si), et
**en ce que** au moins un paramètre variable du signal d'avertissement comprend la cadence d'avertissement, le dispositif de traitement (23) étant adapté pour modifier la cadence d'avertissement après que la temporisation est écoulée, la section d'avertissement (Sa) de chaque période d'avertissement ayant une même durée et le laps de temps de chaque période d'avertissement variant en fonction de la cadence d'avertissement.

2. Système médical (10) selon la revendication 1, dans lequel la cadence d'avertissement est comprise entre 1 Hz et 20 Hz.

3. Système médical (10) selon l'une quelconque des revendications 1 et 2, dans lequel ledit au moins un paramètre variable du signal d'avertissement comprend une fréquence d'avertissement à laquelle chacune des sections d'avertissement est émise, le dispositif de traitement (23) étant adapté pour modifier la fréquence d'avertissement après que la temporisation (T) est écoulée.

4. Système médical (10) selon la revendication 3, dans lequel la fréquence d'avertissement est comprise entre 470 Hz et 2600 Hz.

5. Système médical (10) selon l'une quelconque des revendications 1 à 4, dans lequel ledit au moins un paramètre variable du signal d'avertissement comprend une amplitude d'avertissement, le dispositif de traitement (23) étant adapté pour modifier l'amplitude d'avertissement après que la temporisation (T) est écoulée.

6. Système médical (10) selon l'une quelconque des revendications 1 à 5, dans lequel le dispositif de traitement (23) est adapté pour déterminer une conductivité électrique comme caractéristique électrique, et pour :
- augmenter le paramètre du signal d'avertissement lorsque la conductivité électrique augmente,
- diminuer le paramètre du signal d'avertissement lorsque la conductivité électrique diminue.

7. Système médical (10) selon l'une quelconque des revendications 1 à 11, dans lequel le dispositif de traitement (23) est adapté pour déterminer une résistivité électrique comme caractéristique électrique, et pour :
- augmenter le paramètre du signal d'avertissement lorsque la résistivité électrique diminue,
- diminuer le paramètre du signal d'avertissement lorsque la résistivité électrique augmente.

8. Système médical (10) selon l'une quelconque des revendications 1 à 7, dans lequel le dispositif de traitement (23) est adapté pour maintenir constant ledit au moins un paramètre variable du signal d'avertissement tant que la caractéristique électrique est inférieure à un seuil, et pour faire varier le paramètre du signal d'avertissement lorsque la caractéristique électrique atteint le seuil.

## Patentansprüche

1. Medizinisches System (10), das dazu bestimmt ist, in eine anatomische Struktur (2, 3) eines Patienten einzudringen, wobei die anatomische Struktur (2, 3) Gewebe umfasst, die unterschiedliche Fähigkeiten aufweisen, einen elektrischen Strom zu leiten, wobei das medizinische System (10) umfasst:
- einen Körper (11), der geeignet ist, in die anatomische Struktur (2, 3) einzudringen, wobei der Körper (11) eine Außenfläche (12) aufweist,
- mindestens eine erste Elektrode (16), die eine erste Kontaktfläche (16a) umfasst, die an der Außenfläche (12) des Körpers (11) angeordnet ist, um mit den Geweben der anatomischen Struktur (2, 3) in Kontakt zu kommen,
- mindestens eine zweite Elektrode (17), die eine zweite Kontaktfläche (17a) umfasst, die an der Außenfläche (12) des Körpers (11) angeordnet ist, um in Abstand von der ersten Kontaktfläche (16a) mit den Geweben der anatomischen Struktur (2, 3) in Kontakt zu kommen,
- einen elektrischen Generator (22), der geeignet ist, einen elektrischen Strom (M) zwischen der ersten (16a) und der zweiten (17a) Kontaktfläche anzulegen,
- eine Verarbeitungsvorrichtung (23), die geeignet ist, eine elektrische Eigenschaft zu bestimmen, die für die Fähigkeit des Gewebes der anatomischen Struktur (2, 3) zwischen der ersten (16a) und der zweiten (17a) Kontaktfläche, den elektrischen Strom (M) zu leiten, repräsentativ ist, und ein Warnsignal auszugeben, das der bestimmten elektrischen Eigenschaft entspricht, wobei das Warnsignal mindestens einen in Abhängigkeit von der bestimmten elektrischen Eigenschaft variablen Parameter aufweist,
wobei das Warnsignal intermittierend ist und einen Warntakt aufweist, gemäß dem von einem Benutzer wahrnehmbare Wamabschnitte (Sa) nacheinander mit einer Zeitspanne (Si) zwischen zwei aufeinanderfolgenden Warnabschnitten (Sa) ausgegeben werden,
wobei die Verarbeitungsvorrichtung (23) geeignet ist, eine Variation der elektrischen Eigenschaft zu erkennen und den mindestens einen variablen Parameter des Warnsignals zu variieren, nachdem eine Zeitverzögerung (T) im Anschluss an die Variation der elektrischen Eigenschaft verstrichen ist,
wobei das medizinische System (10) **dadurch gekennzeichnet ist, dass** die Zeitverzögerung (T) gleich einem Teil einer Warnperiode ist, die dem Warntakt entspricht, und einen Warnabschnitt (Sa) und eine Zeitspanne (Si) umfasst, und dadurch, dass mindestens ein variabler Parameter des Warnsignals den Warntakt umfasst, wobei die Verarbeitungsvorrichtung (23) geeignet ist, den Warntakt zu modifizieren, nachdem die Zeitverzögerung verstrichen ist, wobei der Warnabschnitt (Sa) jeder Warnperiode dieselbe Dauer aufweist und die Zeitspanne jeder Warnperiode in Abhängigkeit vom Warntakt variiert.

2. Medizinisches System (10) nach Anspruch 1, wobei der Warntakt im Bereich zwischen 1 Hz und 20 Hz liegt.

3. Medizinisches System (10) nach einem der Ansprüche 1 und 2, wobei der mindestens eine variable Parameter des Warnsignals eine Warnfrequenz umfasst, mit der jeder der Warnabschnitte ausgegeben wird, wobei die Verarbeitungsvorrichtung (23) geeignet ist, die Warnfrequenz zu modifizieren, nachdem die Zeitverzögerung (T) verstrichen ist.

4. Medizinisches System (10) nach Anspruch 3, wobei die Warnfrequenz im Bereich zwischen 470 Hz und 2600 Hz liegt.

5. Medizinisches System (10) nach einem der Ansprüche 1 bis 4, wobei der mindestens eine variable Parameter des Warnsignals eine Warnamplitude umfasst, wobei die Verarbeitungsvorrichtung (23) geeignet ist, die Warnamplitude zu modifizieren, nachdem die Zeitverzögerung (T) verstrichen ist.

6. Medizinisches System (10) nach einem der Ansprüche 1 bis 5, wobei die Verarbeitungsvorrichtung (23) geeignet ist, eine elektrische Leitfähigkeit als elektrische Eigenschaft zu bestimmen, und:
- den Parameter des Warnsignals zu erhöhen, wenn die elektrische Leitfähigkeit zunimmt,
- den Parameter des Warnsignals zu verringern, wenn die elektrische Leitfähigkeit abnimmt.

7. Medizinisches System (10) nach einem der Ansprüche 1 bis 11, wobei die Verarbeitungsvorrichtung (23) geeignet ist, einen spezifischen elektrischen Widerstand als elektrische Eigenschaft zu bestimmen, und:
- den Parameter des Warnsignals zu erhöhen, wenn der spezifische elektrische Widerstand abnimmt,
- den Parameter des Warnsignals zu verringern, wenn der spezifische elektrische Widerstand zunimmt.

8. Medizinisches System (10) nach einem der Ansprüche 1 bis 7, wobei die Verarbeitungsvorrichtung (23) geeignet ist, den mindestens einen variablen Parameter des Warnsignals konstant zu halten, solange die elektrische Eigenschaft kleiner als eine Schwelle ist, und den Parameter des Warnsignals zu variieren, wenn die elektrische Eigenschaft die Schwelle erreicht.

## Claims

1. A medical system (10) intended for penetrating an anatomical structure (2, 3) of a patient, the anatomical structure (2, 3) having tissues presenting different capacities for conducting electric current, said medical system (10) comprising:
- a body (11) suitable for penetrating the anatomical structure (2,3), the body (11) having an outer surface (12),
- at least one first electrode (16) having a first contact surface (16a) arranged on the outer surface (12) of the body (11) so as to come into contact with the tissues of the anatomical structure (2, 3),
- at least one second electrode (17) having a second contact surface (17a) arranged on the outer surface (12) of the body (11) so as to come into contact with the tissues of the anatomical structure (2, 3) at a distance from the first contact surface (16a),
- an electric generator (22) suitable for applying an electric current (M) between the first (16a) and second (17a) contact surfaces,
- a processing device (23) suitable for determining an electrical characteristic representative of the capacity of the tissue of the anatomical structure (2, 3) between the first (16a) and second (17a) contact surfaces to conduct the electric current (M), and for emitting a warning signal corresponding to the determined electrical characteristic, the warning signal having at least one parameter that varies as a function of the determined electrical characteristic,
wherein the warning signal is intermittent and has a warning cadence according to which user-perceptible warning sections (Sa) are emitted successively with a time interval (Si) between two successive warning sections (Sa),
wherein the processing device (23) is adapted to detect a variation of the electrical characteristic and to vary said at least one variable parameter of the warning signal after a time delay (T) following the variation of the electrical characteristic has elapsed,
the medical system (10) being **characterized in that** the time delay is equal to a portion of a warning period corresponding to the warning cadence and comprising one warning section (Sa) and one time interval (Si), and
**in that** at least one variable parameter of the warning signal comprises the warning cadence, the processing device (23) being adapted to modify the warning cadence after the time delay has elapsed, the warning section (Sa) of each warning period having a same duration and the time interval of each warning period varying as a function of the warning cadence.

2. The medical system (10) according to claim 1, wherein the warning cadences is between 1 Hz and 20 Hz.

3. The medical system (10) according to any of claims 1 and 2, wherein said at least one variable parameter of the warning signal comprises a warning frequency at which each of the warning sections is emitted, the processing device (23) being adapted to modify the warning frequency after the time delay (T) has elapsed.

4. The medical system according to claim 3, wherein the warning frequency is between 470 Hz and 2600 Hz.

5. The medical system (10) according to any of claims 1 to 4, wherein said at least one variable parameter of the warning signal comprises a warning amplitude, the processing device being adapted to modify the warning amplitude after the time delay (T) has elapsed.

6. The medical system (10) according to any of claims 1 to 5, wherein the processing device (23) is adapted to determine an electrical conductivity as the electrical characteristic, and to:
- increase the parameter of the warning signal when the electrical conductivity increases,
- decrease the parameter of the warning signal when the electrical conductivity decreases.

7. The medical system (10) according to claim 1, wherein the processing device (23) is adapted to determine an electrical resistivity as the electrical characteristic, and to:
- increase the parameter of the warning signal when the electrical resistivity decreases,
- decrease the parameter of the warning signal when the electrical resistivity increases.

8. The medical system according to any of claims 1 to 7, wherein the processing device (23) is adapted to keep constant said at least one variable parameter of the warning signal as long as the electrical characteristic is below a threshold, and to vary the parameter of the warning signal when the electrical characteristic reaches the threshold.
